(19) Europäisches Patentamt
European Patent Office
Office européen des brevets

(11) **EP 2 438 905 B1**

(12) **EUROPEAN PATENT SPECIFICATION**

(45) Date of publication and mention
of the grant of the patent:
**24.01.2018 Bulletin 2018/04**

(21) Application number: **10783117.4**

(22) Date of filing: **28.05.2010**

(51) Int Cl.:
*A61K 8/39* *(2006.01)*    *A61K 8/03* *(2006.01)*
*A61K 8/31* *(2006.01)*    *A61K 8/34* *(2006.01)*
*A61K 8/42* *(2006.01)*    *A61K 8/73* *(2006.01)*
*A61K 8/86* *(2006.01)*    *A61K 8/891* *(2006.01)*
*A61Q 1/14* *(2006.01)*    *A61Q 19/10* *(2006.01)*
*A61K 8/02* *(2006.01)*    *A61K 8/37* *(2006.01)*
*A61K 8/49* *(2006.01)*

(86) International application number:
**PCT/JP2010/003590**

(87) International publication number:
**WO 2010/140329 (09.12.2010 Gazette 2010/49)**

(54) **TRANSPARENT MULTI-LAYERED LIQUID COSMETIC**

TRANSPARENTES UND MEHRSCHICHTIGES FLÜSSIGKOSMETIKUM

COSMÉTIQUE LIQUIDE MULTICOUCHE TRANSPARENT

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**

(30) Priority: **03.06.2009 JP 2009134019**

(43) Date of publication of application:
**11.04.2012 Bulletin 2012/15**

(73) Proprietor: **Kao Corporation
Chuo-Ku
Tokyo 103-8210 (JP)**

(72) Inventor: **TOMOKUNI, Atsushi
Tokyo 131-8501 (JP)**

(74) Representative: **Hoffmann Eitle
Patent- und Rechtsanwälte PartmbB
Arabellastraße 30
81925 München (DE)**

(56) References cited:
**WO-A2-02/15849        JP-A- 8 099 836
JP-A- 10 101 529        JP-A- 60 078 907
JP-A- 2001 233 729        JP-A- 2002 003 339
JP-A- 2002 241 223        JP-A- 2002 294 084
JP-A- 2004 010 517        JP-A- 2004 217 641
JP-A- 2005 336 089        US-A1- 2004 033 914
US-A1- 2006 079 418**

Remarks:
The file contains technical information submitted after
the application was filed and not included in this
specification

**Description**

TECHNICAL FIELD

The present invention relates to a clear multi-layered liquid cosmetic.

BACKGROUND ART

Multi-layered cosmetics have been used as cleansing agents for removing makeup, or for removing general impurities (see Patent Documents 1 to 4, for example).
Multi-layered cosmetics are separated into multiple layers before use, and are turned into a single-layered emulsion when shaken for use. The emulsified cosmetics may thereafter be applied to the skin or hair, so as to cleanse, typically remove makeup materials or sebum.

RELATED DOCUMENT

[Patent Document]

[Patent Document 1]
Japanese Patent Publication No. JP-A-H08-99836
[Patent Document 2]
Japanese Patent Publication No. JP-A-H10-101529
[Patent Document 3]
Japanese Patent Publication No. JP-A-2004-203764
[Patent Document 4]
Japanese Patent Publication No.JP-A-H11-246348 Further documents are JP 2004-217641 and WO 02/15849 both of which also concern multi-layer make-up remover compositions.

DISCLOSURE OF THE INVENTION

Multi-layered cosmetics preferably satisfy the points described below:

(i) excellent usability such that it may readily be emulsified by shaking, kept emulsified during use by the user, and may quickly separate into multiple layers after use;
(ii) strong cleansing power capable of thoroughly removing makeup materials and so forth;
(iii) refreshing feel to the touch during use without stickiness; and
(iv) stylish appearance of the multi-layered state.

It is, however, difficult to satisfy all of (i) to (iv) by the techniques described in Patent Documents 1 to 4.
The cosmetic described in Patent Document 1 turns into a W/O-type emulsion when shaken, and feels sticky to the user.
While the cosmetics described in Patent Documents 2 and 4 turn into O/W-type when shaken, they contain large amounts of surfactant and stay in stable emulsion, so that they have poor cleansing power, are not quickly separable into multiple layers, and are degraded in appearance. In general, oil will become less interactive to makeup materials when encapsulated by water, and will therefore become difficult to exhibit strong cleansing power. In particular, if the amount of surfactant is large, the emulsified state will stabilize, the cleansing power will degrade, re-separation will be slow, and thereby a white turbid emulsified state will persist. A large surfactant content will also result in a cosmetic that is sticky to the touch during use.
The cosmetic described in Patent Document 3 is configured by an opaque emulsion layer and an aqueous layer when allowed to stand still, and is therefore not stylish and attractive.
Therefore the present invention relates to a clear multi-layered liquid cosmetic according to claim 1 which satisfies all of (i) to (iv) described in the above.
The present inventors have found that a clear multi-layered liquid cosmetic, which ensures sufficient levels of readiness in emulsification when shaken, and quick separability into multiple layers after use, has strong cleansing power exerted on makeup materials, provides a refreshing feel to the touch during use without stickiness, and has an excellent appearance when separated into multiple layers, may be obtained by combining (A) a trace amount of nonionic surfactant, (B) a water-soluble cellulose derivative, (C) an oil, and (D) water.

According to the present invention, there is provided a clear multi-layered liquid cosmetic which includes:

(A) not less than 0.0005% by weight and not more than 1% by weight of a nonionic surfactant configured by a single species of nonionic surfactant, or a mixed surfactant containing two or more species of nonionic surfactants mixed therein, having an HLB of the single species of nonionic surfactant or the mixed surfactant of 5 to 11;
(B) not less than 0.05% by weight and not more than 5% by weight of a water-soluble cellulose derivative;
(C) not less than 10% by weight and not more than 90% by weight of an oil having a viscosity at 30°C of not more than 30 mPa·s; and
(D) water,

wherein the cosmetic is configured to separate into multiple layers when allowed to stand still, and to form an O/W-type emulsion when mixed by shaking and wherein the ratio by weight given by (B)/(A) is not less than 2 and not more than 500. "Clear multi-layered" herein means that the individual layers are not emulsified when allowed to stand still, instead showing a clear multi-layered state. This makes the appearance of the cosmetic stylish.

Since the clear multi-layered liquid cosmetic of the present invention turns into an O/W-type emulsion by shaking, a continuous phase of the clear multi-layered liquid cosmetic becomes water. Therefore, refreshing feel to the touch during use may be obtained. The emulsion quickly separates upon contact with makeup materials, and the oil composing the component (C) can effectively interact with the makeup materials, so that strong cleansing power may be exhibited. The cosmetic is ready to emulsify when shaken, kept emulsified when used by the user, and separates quickly into multiple layers after use.

According to the present invention, an excellent clear multi-layered liquid cosmetic, which satisfies all of the characteristics (i) to (iv) described in the above in a well-balanced manner, may be provided.

In addition, the present invention also provides a method of removing makeup materials, by shaking it for mixing and then applying the above-described clear multi-layered liquid cosmetic, onto made-up skin.

According to the present invention, an excellent clear multi-layered liquid cosmetic, which satisfies all of the required characteristics (i) to (iv) described in the above in a well-balanced manner, may be provided.

DESCRIPTION OF THE EMBODIMENTS

According to the present invention, a clear multi-layered liquid cosmetic which is excellent in usability, such that it separates into multiple layers when allowed to stand still, readily emulsifies when shaken, kept emulsified when used by the user, and quickly separates after use; has strong cleansing power; has refreshing feel to the touch upon use; and has a stylish appearance when separated into multiple layers, may be provided.

Embodiments will be explained below.

The clear multi-layered liquid cosmetic contains at least the component (A) to component (D) below, and is configured to separate into multiple layers when allowed to stand still, and to form an O/W-type emulsion upon being shaken.

(Component (A))

The component (A) is a nonionic surfactant which is configured by a single species of nonionic surfactant, or a mixed surfactant containing two or more species of nonionic surfactants mixed therein, and has an HLB of the single species of nonionic surfactant or the mixed surfactant of 5 to 11.

HLB (Hydrophilic-Lipophilic Balance) represents a ratio of molecular weight of the hydrophilic group portion relative to the total molecular weight of the surfactant, and is determined according to the Griffin's equation if the nonionic surfactant is a polyoxyethylene-based one.

The HLB of the mixed surfactant, configured by two or more species of nonionic surfactants, may be determined as described below.

For a system containing a plurality of nonionic surfactants mixed therein, the mixed-system HLB is obtained by calculating an arithmetical average of the HLB values of the individual nonionic surfactants based on their ratios of mixing.

$$\mathtt{Mixed\text{-}system\ HLB} = \Sigma(\mathtt{HLBx} \times \mathtt{Wx})/\Sigma\mathtt{Wx}$$

HLBx represents an HLB value of nonionic surfactant X.

Wx represents weight (g) of the nonionic surfactant X having a value of HLBx.

The nonionic surfactant composing the component (A) used in the present invention is typically composed of a fatty acid ester having 8 to 22 carbon atoms, or an ether from higher alcohols having 8 to 22 carbon atoms, and has a hydroxy group, amide group, or ethylene oxide group as a hydrophilic functional group. Among them, those having a highly-hydrophilic hydroxy group or amide group, which has a high hydrophilic property,are preferable.

Specific examples include glycerin fatty acid ester, polyglycerin fatty acid ester, polyoxyethylene glycerin fatty acid ester, propylene glycol fatty acid ester, polyoxyethylene polyoxypropylene glycol, sorbitan fatty acid ester, polyoxyethylene sorbitan fatty acid ester, polyoxyethylene castor oil, polyoxyethylene hydrogenated castor oil, fatty acid ester of polyoxyethylene hydrogenated castor oil, polyethylene glycol fatty acid ester, alkylglyceryl ether, alkylpolyglyceryl ether, polyoxyethylene alkyl ether, polyoxyethylene alkyl ether fatty acid ester, alkylalkanolamide, polyoxyethylene alkylamine, and fatty acid alkanolamide.

Among them, those having a plurality of hydrophilic functional groups are preferable from the viewpoint of readiness of emulsification, and in particular those having two or more hydroxyl groups, or those having a hydroxy group and an amide group are preferable.

The nonionic surfactant having two or more hydroxyl groups are exemplified by glycerin mono(fatty acid) ester, diglycerin mono(fatty acid) ester, polyoxyethylene glycerin mono(fatty acid) ester, sorbitan fatty acid ester, polyoxyethylene sorbitan fatty acid ester, alkylglyceryl ether, and alkyldiglyceryl ether.

The nonionic surfactant having a hydroxy group and an amide group are exemplified by fatty acid alkanolamide such as coconut fatty acid-methylmonoethanol amide.

Among these, the nonionic surfactant having two or more hydroxyl groups are excellent in terms of readiness of emulsification of oil.

The glycerin mono(fatty acid) ester is more preferably a monoester formed by glycerin and a fatty acid having 12 to 18 carbon atoms. Specific examples include glycerin monolaurate (HLB5), glycerin monostearate (HLB3), glycerin monoisostearate (HLB3), and glycerin monooleate (HLB3).

The diglycerin mono(fatty acid) ester is more preferably a monoester formed by diglycerin and a fatty acid having 8 to 18 carbon atoms. Specific examples include diglycerin monolaurate (HLB9), diglycerin monostearate (HLB8), diglycerin monoisostearate (HLB8), and diglycerin monooleate (HLB8).

The polyoxyethylene glycerin mono(fatty acid) ester is more preferably a monoester formed by glycerin, a polyoxyethylene having a degree of polymerization of 3 to 30, and a fatty acid having 8 to 18 carbon atoms. Specific examples include polyoxyethylene (6) glycerin monocaprylate/caprate (HLB13), polyoxyethylene (7) glycerin mono(coconut oil fatty acid) ester (HLB11), polyoxyethylene (30) glycerin monostearate (HLB16), polyoxyethylene (6) glycerin monoisostearate (HLB8), polyoxyethylene (8) glycerin monoisostearate (HLB9), and polyoxyethylene (10) glycerin monoisostearate (HLB10).

The sorbitan fatty acid ester is more preferably a monoester formed by sorbitan and a fatty acid having 8 to 18 carbon atoms. Specific examples include sorbitan monolaurate (HLB10), sorbitan monostearate (HLB8), sorbitan monoisostearate (HLB8), and sorbitan monooleate (HLB9).

The polyoxyethylene sorbitan fatty acid ester is more preferably a monoester formed by sorbitan, a polyoxyethylene having a degree of polymerization of 3 to 30, and a fatty acid having 8 to 18 carbon atoms. Specific examples include polyoxyethylene (6) sorbitan monolaurate (HLB13), polyoxyethylene (20) sorbitan monolaurate (HLB17), polyoxyethylene (6) sorbitan monostearate (HLB10), polyoxyethylene (20) sorbitan monostearate (HLB15), polyoxyethylene (6) sorbitan monoisostearate (HLB10), polyoxyethylene (20) sorbitan monoisostearate (HLB15), polyoxyethylene (6) sorbitan monooleate (HLB10), and polyoxyethylene (20) sorbitan monooleate (HLB14).

The alkylglyceryl ether is more preferably a monoether formed by glycerin and a higher alcohol having 8 to 18 carbon atoms. Specific examples include mono(2-ethylhexyl) monoglyceryl ether (HLB7), monolauryl monoglyceryl ether (HLB6), monostearyl monoglyceryl ether (HLB5), monoisostearyl monoglyceryl ether (HLB5), and monooleyl monoglyceryl ether (HLB5).

The alkyldiglyceryl ether is more preferably a monoether formed by diglycerin and a higher alcohol having 8 to 18 carbon atoms. Specific examples include mono(2-ethylhexyl) diglyceryl ether (HLB10), monolauryl diglyceryl ether (HLB9), monostearyl diglyceryl ether (HLB8), monoisostearyl diglyceryl ether (HLB8), and monooleyl diglyceryl ether (HLB8).

Among these, monoglycerin mono(fatty acid) ester, diglycerin mono(fatty acid) ester, alkylglyceryl ether, and alkyldiglyceryl ether are preferable, from the viewpoint of readiness of separation when used.

In particular, from the viewpoints of readiness of manufacturing and preservation stability, the nonionic surfactant composing the component (A) used herein preferably exists as a liquid at 20°C, or as a paste. Specific examples include glycerin monoisostearate (HLB3), glycerin monooleate (HLB3), diglycerin monoisostearate (HLB8), diglycerin monooleate (HLB8), mono(2-ethylhexyl) monoglyceryl ether (HLB7), monolauryl monoglyceryl ether (HLB6), monoisostearyl monoglyceryl ether (HLB5), monooleyl monoglyceryl ether, (HLB5), mono(2-ethylhexyl) (diglyceryl ether (HLB10), monolauryl diglyceryl ether (HLB9), monoisostearyl diglyceryl ether (HLB8), and monooleyl diglyceryl ether (HLB8).

Further from the viewpoint of refreshing feel to the touch upon use, more preferable examples include glycerin monoisostearate (HLB3), diglycerin monoisostearate (HLB8), mono(2-ethylhexyl) monoglyceryl ether (HLB7), monoisostearyl monoglyceryl ether (HLB5), mono(2-ethylhexyl) diglyceryl ether (HLB10), and monoisostearyl diglyceryl ether (HLB8).

The HLB value of the nonionic surfactant, configured by a single species of nonionic surfactant or a mixed surfactant containing two or more species of nonionic surfactants, may be not more than 11, and preferably not more than 10 from the viewpoint of enhancement of lipophilicity. An HLB value is preferably not less than 5.

The component (A) is preferably configured by only nonionic surfactant(s) having HLB value(s) of 5 to 11. By the adjustment, emulsification of the oil composing the component (C) may be promoted, and the emulsified state may more readily be broken upon contact with the makeup materials. The oil composing the component (C) may therefore interact directly with the makeup impurities, and thereby a strong cleansing power may be exhibited.

Content of the component (A) is adjusted to not less than 0.0005% by weight and not more than 1% by weight in the clear multi-layered liquid cosmetic. By adjustment, the clear multi-layered liquid cosmetic may quickly be emulsified when shaken, and may quickly separate into multiple layers after use. On the other hand, by adjusting the content of the component (A) to not more than 1% by weight, the clear multi-layered liquid cosmetic may be less stimulative to the skin. In particular, a content of the component (A) of not less than 0.001% by weight in the clear multi-layered liquid cosmetic is excellent, in view of quick emulsification upon shaking, and quick separation into multiple layers.

The content of the component (A) is preferably not more than 0.29% by weight, and more preferably not more than 0.02% by weight, in the clear multi-layered liquid cosmetic, from the viewpoints of feel to the touch upon use, and separability of the layers.

The clear multi-layered liquid cosmetic of this embodiment preferably contains nothing other than the component (A) as the surfactant.

(Component (B))

The water-soluble cellulose derivative composing the component (B) used in the present invention is a water-soluble product obtained by substituting a part of, or all of, hydroxyl groups of cellulose with other groups, and is particularly preferably a nonionic water-soluble cellulose.

Specific examples include hydroxyalkyl cellulose, alkyl cellulose, and alkylhydroxyalkyl cellulose, which have alkyl groups each having 1 to 5 carbon atoms.

Among these, from the viewpoints of excellence of appearance in the multi-layered separated state, and sustainability of the emulsified state when shaken, those having hydroxyalkyl groups are preferable. More specifically, a single species, or two or more species are preferably selected from hydroxyethyl cellulose, hydroxypropyl cellulose, hydroxypropylmethyl cellulose, hydroxyethylmethyl cellulose, and hydroxypropylethyl cellulose.

From an additional viewpoint of cleansing power enough to thoroughly remove the makeup materials or the like, it is particularly preferable to use hydroxyethyl cellulose, or hydroxypropyl cellulose.

Weight average molecular weight of the component (B) is preferably not less than 10,000 and not more than 900,000, more preferably not more than 400,000, and even more preferably not less than 55,000 and not more than 250,000, from the viewpoint of readiness of emulsification when shaken.

The component (B) is a single species, or two or more species of water-soluble cellulose derivatives, where the content of which is preferably not less than 0.05% by weight and not more than 5% by weight of the entire portion of the clear multi-layered liquid cosmetic.

By adjusting the content to not less than 0.05% by weight, the clear multi-layered liquid cosmetic may quickly be emulsified upon shaking. By adjusting the content to not more than 5.0% by weight, the clear multi-layered liquid cosmetic may quickly be separated into multiple layers after use.

In particular, the content of the component (B) is preferably not more than 3.0% by weight in the clear multi-layered liquid cosmetic, and not less than 0.1% by weight.

The water-soluble cellulose derivative composing the component (B) is supposed to stabilize the emulsified state of the oil composing the component (C) emulsified by the nonionic surfactant composing the component (A), at an appropriate level, and to sustain the emulsified state at least over the duration of use by the user.

The content of other water-soluble polymers may not be more than 0.1% by weight, preferably 0.05% by weight, and more preferably zero. Presence of the other water-soluble polymers may considerably inhibit the readiness of emulsification upon shaking, and the separability.

The ratio of weight of the component (B) and component (A) ((B)/(A)) is not less than 2 and not more than 500, and particularly preferably not less than 2.5 and not more than 200. By adjustment, the clear multi-layered liquid cosmetic may now be readily emulsified when shaken, and may be enhanced in the separability after shaking. In other words, by mixing the component (B) within a predetermined range but more than the component (A), it becomes possible to concurrently achieve an effect of promoting emulsification when shaken and an effect of promoting destroying of emulsification upon contact with the makeup materials as contributed by the component (A), and, stability of emulsification as contributed by the component (B).

(Component (C))

The oil composing the component (C) used in the present invention exists as a liquid at normal temperature, and has a viscosity at 30°C of not more than 30 mPa·s, and preferably not more than 5 mPa·s. The viscosity herein is measured using a BM-type viscometer (from Tokimec Co., Ltd., measurement conditions: rotor No.1, 60 rpm).

This sort of low-viscosity oil is highly permeable into finely-profiled portions covered with makeup materials, exhibits a high ability of solubilizing them, and thereby exhibits a strong cleansing power, which may be effected on oily impurities such as oil-based mascara, lipstick and so forth. The oil is felt less oily, provides a pleasant feel to the touch upon use, and is stable in the emulsified state.

The oil adoptable herein may be those used for general cosmetics, skin cleansing agents and makeup removers, and may be selectable from hydrocarbon oils, ester oils, ether oils, higher alcohols, higher fatty acids, fluorinated oils and silicones.

For example, the higher alcohols are exemplified by straight-chain or branched-chain higher alcohols having alkyl or alkenyl group, and preferably by straight-chain or branched-chain higher alcohols having 8 to 28 carbon atoms and having alkyl or alkenyl group. More specifically, lauryl alcohol, isomyristyl alcohol, isopalmityl alcohol, isostearyl alcohol, and oleyl alcohol are exemplified.

The higher fatty acids are exemplified by straight-chain or branched-chain higher fatty acids having alkyl or alkenyl group, and preferably by branched or unsaturated fatty acids having 8 to 28 carbon atoms and having alkyl or alkenyl group. More specifically, isomyristic acid, isopalmitic acid, isostaric acid, and oleic acid are exemplified.

The hydrocarbon oils are exemplified by squalane, liquid isoparaffin, and liquid paraffin.

The ester oils are exemplified by those formed by botanical oils, or straight-chain or branched-chain saturated or unsaturated fatty acids having 8 to 28 carbon atoms, and straight-chain or branched-chain saturated or unsaturated alcohols having 2 to 28 carbon atoms. More specifically, isononyl isononanoate, isotridecyl isononanoate, isopropyl palmitate, isopropyl myristate, isopropyl isostearate, cetyl 2-ethylhexanoate, octadecyl myristate, neopentyl glycol dicaprate, glycerin tri(2-ethylhexanoate), and glycerin tri(caprylate/caprate) are exemplified.

The ether oils are exemplified by those formed by two species of alcohols selected from straight-chain or branched-chain saturated or unsaturated alcohols having 8 to 28 carbon atoms. More specifically, alkyl-1,3-dimethylbutyl ether, dicaprylyl ether, dicapryl ether, dilauryl ether, and diisostearyl ether are exemplified.

Silicones are exemplified by dimethylpolysiloxane, methylphenylpolysiloxane, cyclomethicone, dimethicone, trisiloxane methyltrimethicone, and ethyltrisiloxane.

The fluorinated oils are exemplified by fluoropolyether, and perfluoroalkylether silicone.

Among these, the hydrocarbon oils, ester oils, ether oils, and silicones are more preferable from the viewpoint of cleansing power for removing makeup materials, and pleasant feel to the touch upon use. Specific examples categorized thereto include hydrocarbons such as dodecane, tetradecane, hexadecane, isododecane, isohexadecane, and isoeicosane; ester oils such as isononyl isononanoate, isopropyl palmitate, isopropyl myristate, isopropyl isostearate, cetyl 2-ethylhexanoate, neopentyl glycol dicaprate, glycerin tri(caprylate/caprate), and dicaprylyl carbonate; ether oils such as alkyl-1,3-dimethylbutyl ether, dicaprylyl ether, and dicapryl ether; and silicones having molecular weights of 100 to 500 such as methylpolysiloxane, decamethylcyclopentasiloxane, dodecamethylhexacyclosiloxane, and octamethylcyclotetrasiloxane.

From the viewpoints of moderating the oiliness to adjust appropriate feel to the touch upon use, and stability of the emulsified state, the component (C) preferably has a viscosity at 25°C of not more than 10 mPa·s. Specific examples include isododecane (viscosity: 1 mPa·s), isohexadecane (viscosity: 4 mPa·s), isopropyl palmitate (viscosity: 10 mPa·s), isopropyl myristate (7 mPa·s), dicaprylyl carbonate (8 mPa·s), dicaprylyl ether (5 mPa·s), decamethylcyclopentasiloxane (viscosity: 4 mPa·s), dodecamethylhexacyclosiloxane (viscosity: 8Pa·s), trisiloxane (viscosity: 1 mPa·s), dimethicone (viscosity: ≤10 mPa·s), methyltrimethicone (viscosity: 2 mPa·s), and ethyltrisiloxane (viscosity: 1 mPa·s).

More preferable examples include isododecane, isohexadecane, dodecamethylhexacyclosiloxane, decamethylcyclopentasiloxane, trisiloxane, dimethicone, methyltrimethicone, and ethyltrisiloxane.

The component (C) preferably contains not less than 50% of the low-viscosity oil having a viscosity of not more than 10 mPa·s, from the viewpoint of enhancing the cleansing power.

The component (C) may be configured by a single species, or two or more species, wherein the content of which is not less than 10% by weight, and not more than 90% by weight of the clear multi-layered liquid cosmetic. In particular, the content is adjusted to not less than 14% by weight and preferably not less than 15% by weight, and not more than 59% by weight and preferably not more than 50% by weight, in view of providing smooth washability and refreshing feel to the touch upon use, while ensuring a sufficient level of cleansing power.

(Component (D))

The component (D) is water, and preferably is a purified water. The content of water is preferably not less than 9% by weight, and not more than 85% by weight of the clear multi-layered liquid cosmetic, from the viewpoint of feel to the touch upon use and cleansing power. In particular, a content of not less than 40% by weight, particularly not less than 49% by weight, and not more than 80% by weight is preferable.

Since the nonionic surfactant composing the component (A) in the present invention is slightly lipophilic having an HLB value of 5 to 11, it can quickly adsorb at the interface between the oil layer and the aqueous layer, so as to reduce the interfacial tension. By virtue of this effect, the clear multi-layered liquid cosmetic can promote dispersion of the oil which composes the component (C), quickly into water which composes the component (D), when shaken.

The state having the component (C) emulsified into water which composes the component (D) may be sustained by the emulsifying action of the water-soluble cellulose derivative which composes the component (B). By virtue of these effects, the article of the present invention may sustain the emulsified state over a short duration in which the user uses the inventive article. In short, the effects of the present invention is supposed to be mainly ascribable to the effect of the water-soluble cellulose derivative which composes the component (B), exerted so as to stabilize the emulsified state, whereas the nonionic surfactant which composes the component (A), the content of which is only as small as not more than 1% by weight, is supposed to assist the emulsification.

On the other hand, when the cosmetic of the present invention, emulsified by shaking, is brought into contact with the makeup materials, the component (A) quickly adsorbs onto the makeup materials having hydrophobic surface. This is because the HLB value of the nonionic surfactant, which composes the component (A), is adjusted to 5 to 11, so as to make it slightly lipophilic. Accordingly, the emulsified state is supposed to be abruptly broken, the component (C) is allowed to contact with the makeup materials, and thereby the effect of cleansing may be exhibited.

According to the above-described mechanism, the component (C) exhibits a strong ability of cleansing against the makeup materials, despite being surrounded by the component (D) to provide an O/W-type emulsion.

As described in the above, the clear multi-layered liquid cosmetic of the present invention produces a stable O/W-type emulsion by shaking, having water as the continuous phase, so that refreshing feel to the touch upon use may be obtained.

Moreover, since the content of the component (A) is adjusted to not more than 1% by weight, the clear multi-layered liquid cosmetic may relatively quickly separate into multiple layers (two layers, for example) after use.

(Component (E))

The clear multi-layered liquid cosmetic of this embodiment preferably contains a water-miscible solvent described below, as the component (E). By containing a water-miscible solvent, the aqueous layer and the oil layer may be more readily miscible when the clear multi-layered liquid cosmetic is shaken.

As the water-miscible solvent, a single species, or two or more species selected from:

(E1) a compound having two or more oxypropylene groups and hydroxyl groups in the molecule thereof, and having a value of (the number of oxypropylene groups/the number of hydroxyl groups) of not more than 5;
(E2) a monohydric alcohol having 2 to 6 carbon atoms; and
(E3) a dihydric alcohol having 2 to 6 carbon atoms, may be used.

(E1) is exemplified by tripropylene glycol, polypropylene glycol (7), polyoxypropylene (3) monoglyceryl ether, polyoxypropylene (6) monoglyceryl ether, polyoxypropylene (9) monoglyceryl ether, polyoxypropylene (9) diglyceryl ether, polyoxypropylene (14) diglyceryl ether, polyoxyethylene (2) polyoxypropylene (2) butyl ether, and polyoxyethylene (5) polyoxypropylene (5) butyl ether.

(E2) is exemplified by ethanol, propanol, isopropanol, butanol, and isobutanol.

(E3) is exemplified by ethylene glycol, propylene glycol, dipropylene glycol, isoprene glycol, 1,3-butylene glycol, 1,2-pentanediol, and hexylene glycol.

Among these, polyoxypropylene (3) monoglyceryl ether, polyoxypropylene (9) diglyceryl ether, ethanol, propylene glycol, dipropylene glycol, isoprene glycol, and 1,3-butylene glycol are preferable, because they can provide refreshing feel to the touch upon use, readily emulsify when shaken, and can keep the emulsified state when used by the user.

Content of the component (E) is adjusted to not less than 1% by weight and not more than 20% by weight, and preferably not less than 5% by weight and not more than 10%, in view of keeping the emulsified state.

(Other Components)

In addition to the above-described component (A) to component (D), and component (E), the clear multi-layered liquid cosmetic may contain a drug component, moisturizer, colorant, antiseptic, fragrance, and so forth.

The clear multi-layered liquid cosmetic of this embodiment preferably contains not so much inorganic salt, wherein the content thereof is preferably less than 1.0% by weight, for example. The content is further preferably not more than 0.1% by weight, even further preferably not more than 0.03% by weight, and even further preferably zero.

The inorganic salt is exemplified by water-soluble salts which dissolve into water to dissociate into ions, such as sodium chloride, sodium sulfate, magnesium sulfate, sodium carbonate, calcium carbonate, sodium hydrogen phosphate, sodium dihydrogen phosphate, and sodium sulfite.

The cosmetic more preferably also contains salts other than not so much inorganic salt, wherein the total content of salts including the inorganic salt is preferably not more than 0.1% by weight, more preferably not more than 0.03% by weight, and even more preferably zero.

The clear multi-layered liquid cosmetic also preferably contains not so much organic acid, wherein the content is preferably less than 1.0% by weight. The content is more preferably not more than 0.1% by weight, even more preferably not more than 0.03% by weight, and even more preferably zero.

The organic acid is exemplified by citric acid, malic acid, succinic acid, lactic acid, and tartaric acid.

Only such small contents of the above-described salts and organic acids are preferable in view of touch of use. In particular, a mild feel to the touch upon use may be obtained, when the cosmetic is applied around the eyes and the mouth.

The clear multi-layered liquid cosmetic of the present invention separates into multiple layers (two layers of oil layer and aqueous layer, for example) when allowed to stand still. The individual layers are clear, and are not emulsified with each other.

The user uses the clear multi-layered liquid cosmetic of the present invention, by shaking it several times. More specifically, the user grips a container of the clear multi-layered liquid cosmetic, and shakes the container 5 to 20 times, at a stroke range of approximately 10 to 30 cm, so as to emulsify the cosmetic which separates into multiple layers. The thus-obtained emulsion is an O/W-type emulsion. The emulsion is used as a cleansing agent spread over the made-up skin, so as to remove the makeup materials. The clear multi-layered liquid cosmetic is kept in the emulsified state during use by the user. After the elapse of a predetermined length of time, the clear multi-layered liquid cosmetic separates again into multiple layers.

While the emulsion may be applied to the skin by hand, it is more preferable to use the emulsion in such a way that the cosmetic is applied onto the made-up skin while being impregnated into cleansing goods such as woven fabric or non-woven fabric, so as to keep the cosmetic emulsified and to more effectively remove the makeup materials.

Note that the present invention is not limited by the above-describe embodiments.

While the clear multi-layered liquid cosmetic in the above-described embodiments were separated into two layers, it may be separated into three or more layers without limitation. The individual layers may be enhanced aesthetically, by coloring using pigments or dyes, so as to allow the user to enjoy a change of color upon mixing.

"Clear" herein means, as described in the paragraphs below for explaining the evaluation method in Examples, a state that the obtained cosmetic is placed in a 100-ml screw-capped glass tube 3 cm in diameter, through which letters on paper placed behind the tube can be read.

[Examples]

Next, Examples of the present invention, and Comparative Examples will be explained.

Clear multi-layered liquid cosmetics were prepared according to the compositions listed in Table 1 to Table 4. More specifically, the water layer components (the components (B), (D), and additionally the component (E) in some Examples and Comparative Examples) heated to 80°C were optionally added with antiseptics and a pH adjusting agent, and the mixtures were cooled down to room temperature while stirring. The oil layer components (the components (A), (C)) were then added while stirring, together with a colorant and a fragrance in some Examples and Comparative Examples, to thereby obtain clear multi-layered liquid cosmetics.

The clear multi-layered liquid cosmetics herein appeared to be separated into two layers which are clear without causing emulsification.

The contents of the individual compositions in Tables are given in % by weight.

Each component (C) used in Examples and Comparative Examples had a viscosity at 30°C of not more than 30 mPa·s. The viscosity was measured using a BM-type viscometer (from Tokimec Co., Ltd., measurement conditions: rotor No.1, 60 rpm).

Each component (A) used in Examples appeared as a liquid or paste at 20°C.

Of the component (A) used in Examples, coconut oil fatty acid N-methyl monoethanolamide has an amide group and a hydroxy group, whereas the others have two or more hydroxyl groups.

The columns showing HLB in mixed state in Tables 1 to 4 represent HLB values of the component (A).

(Evaluation Methods)

1. Appearance

Each of the obtained cosmetics was placed in a sample container, the content was shaken, the appearance of the content after causing separation was visually observed, and clearness of the individual layers were evaluated. The clearness was evaluated by placing a paper, having letters printed thereon, behind the sample container in contact therewith, and by observing the paper from the front.

> sample container: 100-ml screw-capped glass tube 3 cm in diameter
> clear: letters behind the sample container are readable; and
> unclear: letters behind the sample container are unreadable.

2. Emulsion Type

Each cosmetic emulsified by shaking was sucked using a dropper, and a single droplet was gently dropped into water contained in a 200-ml beaker. Changes of the emulsion were observed, and evaluated as follows;

> O/W-type: the emulsion diffused and vanished into water; and
> W/O-type: the emulsion floated in the upper part of the water without vanishing thereinto.

3. Readiness of Emulsification

50 g each of the prepared cosmetic was placed into a 110-ml glass container, and the container was closed with a lid. The glass container was firmly grasped, and then shaken at a stroke range of approximately 30 cm, while keeping a pace of twice a second, ten times in total. The cosmetic was allowed to stand still, the appearance immediately thereafter was visually observed, and evaluated according to the following criteria, wherein A and B are acceptable levels for practical use:

> A: uniformly emulsified;
> B: nearly uniformly emulsified;
> C: separation of either layer observed; and
> D: almost not emulsified.

4. Separability

The samples evaluated in the above with respect to "readiness of emulsification" were then subjected to measurement for determining the time necessary for recovering the multi-layered state, with almost no visible emulsion, after being allowed to stand still, and were evaluated according to the criteria below, wherein A and B are acceptable levels for practical use:

> A: the time that the multi-layered state was recovered was 2 minutes or longer and shorter than 1 hour;
> B: the time that the multi-layered state was recovered was 1 minute or longer and shorter than 2 minutes, or one hour or longer and shorter than 24 hours;
> C: the time that the multi-layered state was recovered was shorter than 1 minute, or 24 hours or longer and shorter than 2 days;
> D: the time that the multi-layered state was recovered was 2 days or longer.

5. Cleansing Power

For the purpose of evaluating the cleansing power, 0.005 g of a waterproof mascara (Maybelline Wonder Curl Mascara Waterproof, from L'Oreal) was uniformly spread over 1.4-cm-diameter circle on artificial leather (urethane, from Okamoto Kasei K.K.), and dried by allowing it to stand for 12 hours. Next, 50 g of each of the formulated cosmetics was placed into a 110-ml glass container, and the container was closed with a lid. The glass container was firmly grasped, and then shaken at a stroke range of approximately 30 cm, while keeping a pace of twice a second, ten times in total, 1.5 g of the shaken cosmetic was then put on cosmetic cotton (Silcot, from Unicharm Corporation), the cosmetic cotton containing the cosmetic was lightly pressed onto the waterproof mascara spread on the artificial leather, and then kept for 10 seconds. The waterproof mascara was then lightly wiped off with a cosmetic cotton. The 10-second pressing and wiping were repeated until the waterproof mascara is almost completely wiped off by visual observation, and the cleansing power was evaluated according to the criteria below, wherein A and B are acceptable levels for practical use:

A: the waterproof mascara was almost completely wiped off by the operation repeated three or smaller number of times;
B: the waterproof mascara was almost completely wiped off by the operation repeated four or five times;
C: the waterproof mascara was almost completely wiped off by the operation repeated six to ten times; and
D: the waterproof mascara could not be wiped off by the operation repeated ten times.

6. Feel to the Touch upon Use

The feel to the touch upon use was evaluated by a method described next. Fifty grams of the prepared cosmetic was placed into a 110-ml glass container, and the container was closed with a lid. The glass container was firmly grasped, and then shaken at a stroke range of approximately 30 cm, while keeping a pace of twice a second, ten times in total, and 1.5 g of the shaken cosmetic was put on cosmetic cotton (Silcot, from Unicharm Corporation). Ten expert panelists lightly wiped their skin around the eyes five times with the cosmetic cotton containing the cosmetic, and gave sensory evaluation according to the criteria below:

A: refreshing feel to the touch felt by 8 or more panelists;
B: refreshing feel to the touch felt by 7 or 6 panelists;
C: refreshing feel to the touch felt by 5 or 4 panelists; and
D: refreshing feel to the touch felt by 3 or smaller number of panelists.

Results of Examples and Comparative Examples are shown in Table 1 to Table 4.

[Table 1]

| Purpose of mixing | Materials | Manufacturer | Component | Ex. 1 | Ex. 2 | Ex. 3 | Ex. 4 | EX. 5 | Ex. 6 |
|---|---|---|---|---|---|---|---|---|---|
| (Component A) Nonionic surfactant (5≤HLB≤11, in mixed state) | Cosmol 41V (HLB=8) | Nisshin oillio Group, Ltd. | Diglycerin monoisostearate | 0.040 | 0.050 | 0.020 | 0.005 | | |
| | Penetol GE-IS (HLB=5) | KAO Corporation | Monoglycerin isostearyl ether | | | | | | 0.020 |
| | Emalex GWIS 110 (HLB=10) | Nihon Emulsion Co., Ltd. | PEG-10 glyceryl isoatereate | | | | | | |
| | Aminone C-11S (HLB=10) | KAO Corporation | Coconut fatty acid N-methylmonoethanol amide | | | | | 0.020 | |
| | Rheodol TW-S120V (HLB=11) | KAO Corporation | Polyoxyethylene (20) sorbitan stearate | | | | | | |
| | Nikkol TI-10VK (HLB=15) | Nikko Chemicals Co., Ltd. | Polyoxyethylene (20) sorbitan isostearate | | | | | | |
| (Component B) Water-soluble cellulose derivative | HEC DATCEL SE400 (MW=250000) | Daicel Chemical Industries, Ltd. | Hydroxyethyl cellulose | 0.600 | | 0.500 | 0.500 | 0.500 | 0.50 |
| | HPC-L (MW=55000) | Nippon Soda Cho., Ltd. | Hydroxypropyl cellulose | | 0.500 | | | | |
| (Component C) Oil | Marukasol R (1 mPa·s) | Maruzen Petrochemical Co., Ltd. | Isododecane | 20.000 | 25.000 | 10.000 | 5.000 | 10.000 | 14.000 |
| | IP Solvent 2028MU (3 mPa·s) | Idemitsu Kosan Co., Ltd. | Liquid Isoparaffin | | | | 15.000 | | 10.000 |
| | Silicone TSA405A (4 mPa·s) | Momentive Performance Materials Japan LLC | Cyclomethicone | 19.960 | 24.950 | 20.000 | | | 14.000 |
| | Silicone KF-96L-5CS (5 mPa·s) | Shin-Etsu Chemical Co., Ltd. | Dimethicone | | | | 10.000 | 20.000 | |
| | Coconard MT (26 mPa·s) | KAO Corporation | Glyceryl tri(capryrate/caprate) | | | | | | |

(continued)

| Purpose of mixing | Materials | Manufacturer | Component | Ex. 1 | Ex. 2 | Ex. 3 | Ex. 4 | EX. 5 | Ex. 6 |
|---|---|---|---|---|---|---|---|---|---|
| (Component D) Water | Purified water | | Purified water | 59.400 | 49.500 | 60.0489 | 62.0639 | 63.8999 | 56.0799 |
| (Component E) water- | SY-DP9 | Sakamoto Yakuhin Kogyo Co., Ltd. | PPG (9) diglyceryl | | | 4.00 | 2.00 | | 0.00 |
| miscible solvent | 1,3-Butylene glycol | Kyowa Hakko Chemical Co., Ltd. | BG | | | 5.00 | 5.00 | 5.00 | 5.00 |
| | Tokiwa Hisolve EPH | Toho Chemical Industry Co., Ltd. | Phenoxy ethanol | | | 0.30 | 0.30 | 0.30 | 0.30 |
| Antiseptic | Methyl paraoxybenzoate | API Corporation | Paraben | | | 0.10 | 0.10 | 0.10 | 0.10 |
| Colorant | Green No.3 | Kishi Kasei Co., Ltd. | Green No.3 | | | 0.0001 | 0.0001 | 0.0001 - | 0.0001 |
| pH adjusting agent | Sodium hydrogen phosphate | Taihei Chemical Industrial Co., Ltd. | Disodium phosphate | | | 0.030 | 0.030 | | |
| Fragrance | U-YCFL-1 | | Fragrance | | | 0.001 | 0.001 | | |
| | Total | | | 100.00 | 100.00 | 100.00 | 100.00 | 100.00 | 100.00 |
| Surfactant | Total composition (%) | | | 0.040 | 0.050 | 0.020 | 0.005 | 0.200 | 0.020 |
| | HLB | | | 8 | 8 | 8 | 8 | 10 | 5 |
| (Component B)/(Component A) | | | | 15.0 | 10.0 | 25.0 | 100.0 | 2.5 | 25.0 |
| Evaluation | 1. Appearance | | | Clear | Clear | Clear | Clear | Clear | Clear |
| | 2. Emulsion type | | | O/W | O/W | O/W | O/W | O/W | O/W |
| | 3. Readiness of emulsification | | | B | B | A | A | A | B |
| | 4. Separability | | | A | A | A | A | B | A |
| | 5. Cleansing power | | | B | B | A | A | A | A |
| | 6. Feel to the Touch upon use | | | A | A | A | A | A | A |

[Table 2]

| Purpose of mixing | Materials | Manufacturer | component | Ex. 7 | Ex. 8 | Ex. 9 | Ex. 10 | Comp. Ex. 1 | Comp Ex. 2 |
|---|---|---|---|---|---|---|---|---|---|
| (Component A) Nonionic surfactant (5≤HLB≤11, in mixed state) | Cosmol 41V (HLB=8) | Nisshin oillio Group, Ltd. | Diglycerin monoisostearate | 0.0005 | 0.001 | | | 0.0002 | |
| | Penetol GE-IS (HLB=5) | KAO Corporation | Monoglycerin isostearyl ether | | | | | | |
| | Emalex GWIS-110 (HLB=10) | Nihon Emulsion Co., Ltd. | PEG-10 glyceryl isosteareate | | | 0.290 | 1.000 | | 2.000 |
| | Aminone C-11S (HLB=10) | KAO Corporation | Coconut fatty acid N-methylmonoethanol amide | | | | | | |
| | Rheodol TW-S120V (HLB-11) | KAO Corporation | Polyoxyethylene (20) sorbitan stearate | | | | | | |
| | Nikkol TI-10VK (HLB=15) | Nikko Chemicals Co., Ltd. | Polyoxyethylene (20) sorbitan isostearate | | | | | | |
| (Component B) Water-soluble cellulose derivative | HEC DAICEL SE400 (MW=250000) | Daicel Chemical Industries, Ltd. | Hydroxyethyl cellulose | 0.200 | 0.200 | 2-00 | 2.000 | 0.200 | 2.00 |
| | HPC-L (MW=55000) | Nippon Soda Co., Ltd. | Hydroxypropyl cellulose | | | | | | |
| (Component C) Oil | Marukasol R (1 mPa·s) | Maruzen Petrochemical Co., Ltd. | Isododecane | 20.000 | 20.000 | 6.000 | 6.000 | 20.000 | 6.000 |
| | IP Solvent 2028MU (3 mPa·s) | Idemitsu Kosan Co., Ltd. | Liquid Isoparaffin | | | | | | |
| | Silicone TSA405A (4 mPa·s) | Momentive Performance Materials Japan LLC | Cyclomethicone | 30.000 | 29.999 | 8.710 | 8.000 | 30.000 | 7.000 ' |
| | Silicone KF-96L-5CS (5 mPa·s) | Shin-Etsu Chemical Co., Ltd. | Dimethicone | | | | | | |
| | Coconard MT (26 mPa·s) | KAO Corporation | Glyceryl tri(capryrate/ caprate) | | | | | | |

(continued)

| Purpose of mixing | Materials | Manufacturer | component | Ex. 7 | Ex. 8 | Ex. 9 | Ex. 10 | Comp. Ex. 1 | Comp Ex. 2 |
|---|---|---|---|---|---|---|---|---|---|
| (Component D) Water | Purified water | | Purified water | 49.800 | 49.800 | 83.000 | 83.000 | 49.800 | 83.000 |
| (Component E) Water-miscible solvent | SY-DP9 | Sakamoto Yakuhin Kogyo Co., Ltd. | PPG (9) diglyceryl | | | | | | |
| | 1,3-Butylene glycol | Kyowa Hakko Chemical Co., Ltd. | BG | | | | | | |
| Antiseptic | Tokiwa Hisolve EPH | Toho Chemical Industry Co., Ltd. | Phenoxy ethanol | | | | | | |
| | Methyl paraoxybenzoate | API Corporation | Paraben | | | | | | |
| Colorant | Green No.3 | Kishi Kasei Co., Ltd. | Green No.3 | | | | | | |
| pH adjusting agent | Sodium hydrogen phosphate | Taihei Chemical Industrial Co., Ltd. | Disodium phosphate | | | | | | |
| Fragrance | U-YCFL-1 | | Fragrance | | | | | | |
| | Total | | | 100.00 | 100.00 | 100.00 | 100.00 | 100.00 | 100.00 |
| Surfactant | Total composition (t) | | | 0.001 | 0.001 | 0.290 | 1.000 | 0.0002 | 2.000 |
| | HLB | | | 8 | 8 | 10 | 10 | 8 | 10 |
| (Component B)/(Component A) | | | | 400.0 | 200.0 | 6.9 | 2.0 | 1000.0 | 1.0 |
| Evaluation | 1. Appearance | | | Clear | Clear | Clear | Clear | Clear | Opaque |
| | 2. Emulsion type | | | O/W | O/W | O/W | O/W | O/W | O/W |
| | 3. Readiness of emulsification | | | B | B | A | A | C | A |
| | 4. Separability | | | B | A | B | B | C | C |
| | 5. Cleansing power | | | B | A | A | B | C | C |
| | 6. Feel to the touch upon use | | | A | A | A | B | A | C |

[Table 3]

| Purpose of mixing | Materials | Manufacturer | component | Ex. 11 | Ex. 12 | Ex. 13 | Ex. 14 | Comp. Ex. 3 | Comp. Ex. 4 |
|---|---|---|---|---|---|---|---|---|---|
| (Component A) Nonionic surfactant (5≤HLB≤11. in mixed state) | Cosmol 41V (HLB=8) | Nisshin Oillio Group, Ltd. | Diglycerin monoisostearate | | | 0.020 | 0.020 | | 0.010 |
| | Penetol GE-IS (HLB=5) | KAO Corporation | Monoglycerin isostearyl ether | | | | | | |
| | Emalex GWIS-110 (HLB=10) | Nihon Emulsion Co., Ltd. | PEG-10 glyceryl isosteareate | 0.010 | 0.010 | | | 0.010 | |
| | Aminone C-11S (HLB=10) | KAO Corporation | Coconut fatty acid N-methylmonoethanol amide | | | | | | |
| | Rheodol TW-S120V (HLB=11) | KAO Corporation | Polyoxyethylene (20) sorbitan stearate | | | | | | |
| | Nikkol TI-10VK (HLB=15) | Nikko Chemicals Co., Ltd. | Polyoxyethylene (20) sorbitan isostearate | | | | | | |
| (Component B) Water-soluble cellulose derivative | HEC DAICEL SE400 (MW=250000) | Daicel Chemical Industries, Ltd. | hydroxyethyl cellulose | 0.050 | 0.10 | 3.000 | 5.000 | 0.01 | 7.000 |
| | HPC-L (MK=55000) | Nippon Soda Co., Ltd. | Hydroxypropyl cellulose | | | | | | |
| (Component C) Oil | Marukasol R (1 mPa·s) | Maruzen Petrochemical Co., Ltd. | Isododecane | 20.000 | 20.000 | 6.000 | 6.000 | 20.000 | 6.000 |
| | IP Solvent 2028MU (3 mPa·s) | Idemitsu Kosan Co., Ltd. | Liquid Isoparaffin | | | | | | |
| | Silicone TSA405A (4 mPa·s) | Momentive Performance Materials Japan LLC | Cyclomethicone | 29.990 | 29.990 | 8.980 | 8.980 | 29.990 | 8.990 |
| | Silicone KF-96L-5CS (5 mPa·s) | Shin-Etsu Chemical Co., Ltd. | Dimethicone | | | | | | |
| | Coconard MT (26 mPa·s) | KAO Corporation | Glyceryl tri(capryrate/ caprate) | | | | | | |

EP 2 438 905 B1

(continued)

| Purpose of mixing | Materials | Manufacturer | component | Ex. 11 | Ex. 12 | Ex. 13 | Ex. 14 | Comp. Ex. 3 | Comp. Ex. 4 |
|---|---|---|---|---|---|---|---|---|---|
| (Component D) Water | Purified water | | Purified water | 49.9500 | 49-900 | 82.000 | 80.000 | 49.9900 | 78.000 |
| (Component E) Water-miscible solvent | SY-DP9 | Sakamoto Yakuhin Kogyo Co., Ltd. | PPG (9) diglyceryl | | | | | | |
| | 1,3-Butylene glycol | Kyowa Hakko Chemical Co., Ltd. | BG | | | | | | |
| Antiseptic | Tokiwa Hisolve EPH | Toho Chemical Industry Co., Ltd. | Phenoxy ethanol | | | | | | |
| | Methyl paraoxybenzoate | API Corporation | Paraben | | | | | | |
| Colorant | Green No.3 | Kishi Kasei Co., Ltd. | Green No.3 | | | | | | |
| pH adjusting agent | Sodium hydrogen phosphate | Taihei Chemical Industrial Co., Ltd. | Disodium phosphate | | | | | | |
| Fragrance | U-YCFL-1 | | Fragrance | | | | | | |
| | Total | | | 100.00 | 100.00 | 100.00 | 100.00 | 100.00 | 100.00 |
| Surfactant | Total composition (%) | | | 0.010 | 0.010 | 0.020 | 0.020 | 0.010 | 0.010 |
| | HLB | | | 10 | 10 | 8 | 8 | 10 | 8 |
| (Component B)/(Component A) | | | | 5.0 | 10.0 | 150.0 | 250.0 | 1.0 | 700.0 |
| Evaluation | 1. Appearance | | | Clear | Clear | Clear | Clear | Clear | Clear |
| | 2. Emulsion type | | | O/W | O/W | O/W | O/W | O/W | O/W |
| | 3. Readiness of emulsification | | | B | B | A | B | C | C |
| | 4. Separability | | | B | B | A | B | C | C |
| | 5. Cleansing power | | | B | A | A | B | C | C |
| | 6. Feel to the touch upon use | | | A | A | A | A | A | A |

EP 2 438 905 B1

[Table 4]

| Purpose of mixing | Materials | Manufacturer | Component | Ex. 15 | Ex. 16 | Ex. 17 | Ex. 18 | Ex. 19 | Ex. 20 | Comp. Ex. 5 |
|---|---|---|---|---|---|---|---|---|---|---|
| (Component A) Non-ionic surfactant (5≤HLB≤11, in mixed state) | Nikkol TI-10VK (HLB=15) | Nikko Chemicals Co., Ltd. | Polyoxyethylene (20) sorbitan isostearate | | | | 0.04 | | | |
| | Rheodol TW-S120V (HLB=11) | KAO Corporation | Polyoxyethylene (20) sorbitan stearate | | | 0.04 | | | | |
| | Aminone C-11S (HLB=10) | KAO Corporation | Coconut fatty acid N-methylmonoethanol amide | | 0.200 | | | | | |
| | Emalex GWIS-110 (HLB=10) | Nihon Emulsion Co., Ltd. | PEG-10 glyceryl isosteareate | 0.025 | | | | | | |
| | Cosmol 41V (HLB=8) | Nisshin Oillio Group, Ltd. | Diglycerin monoisoatearate | 0.025 | 0.010 | 0.100 | 0.100 | 0.010 | 0.010 | |
| | Penetol GE-IS (HLB=5) | KAO Corporation | Monoglycerin isostearyl ether | | | | | | | |
| | Excel O-95R (HLB=4) | KAO Corporation | Glycerin stearate | | | | | | | 0.100 |
| (Component B) Water-soluble cellulose derivative | HEC DAICEL SE400 (MW=250000) | Daicel Chemical Industries, Ltd. | Hydroxyethyl cellulose | 0.500 | 0.50 | 0.50 | 0.50 | 0.25 | 0.50 | 0.50 |
| | HPC-L (MW=55000) | Nippon Soda Co., Ltd. | Hydroxypropyl cellulose | | | | | 0.25 | | |

(continued)

| Purpose of mixing | Materials | Manufacturer | Component | Ex. 15 | Ex. 16 | Ex. 17 | Ex. 18 | Ex. 19 | Ex. 20 | Comp. Ex. 5 |
|---|---|---|---|---|---|---|---|---|---|---|
| (Component C) Oil | Marukasol R (1 mPa·s) | Maruzen Petrochemical Co., Ltd. | Isododecane | 20.000 | 20.000 | 10.000 | 10.000 | 10.000 | | 10.000 |
| | IP Solvent2028MU (3 mPa·s) | Idemitsu Kosan Co., Ltd. | Liquid Isoparaffin | | | | | | | |
| | Silicone TSA405A (4 mPa·s) | Momentive Performance Materials Japan LLC | Cyclomethicone | 29.950 | 29.990 | 19.900 | 19.900 | 19.900 | 19.900 | 19.900 |
| | Silicone KF-96L-5CS (5 mPa·s) | Shin-Etsu Chemical Co., Ltd. | Dimethicone | | | | | | | |
| | Coconard MT (26 mPa·s) | KAO Corporation | Glyceryl tri(capryrate/caprate) | | | | | | 10.000 | |
| (Component D) Water | Purified water | | Purified water | 49.500 | 49.300 | 64.060 | 64.060 | 69.590 | 69.590 | 69.500 |
| (Component E) Water-miscible solvent | SY-DP9 | Sakamoto Yakuhin Kogyo Co., Ltd. | PPG (9) diglyceryl | | | | | | | |
| | 1,3-Butylene glycol | Kyowa Hakko Chemical Co., Ltd. | BG | | | 5.00 | 5.00 | | | |
| Antiseptic | Tokiwa Hisolve EPH | Toho Chemical Industry Co., Ltd. | Phenoxy ethanol | | | 0.30 | 0.30 | | | |
| | Methyl paraoxybenzoate | API Corporation | Paraben | | | 0.10 | 0.10 | | | |
| Colorant | Green No.3 | Kishi Kasei Co., Ltd. | Green No.3 | | | 0.0001 | 0.0001 | | | |
| pH adjusting agent | Sodium hydrogen phosphate | Taihei Chemical Industrial Co., Ltd. | Disodium phosphate | | | | | | | |
| Fragrance | U-YCFL-1 | | Fragrance | | | | | | | |
| | Total | | | 100.00 | 100.00 | 100.00 | 100.00 | 100.00 | 100.00 | 100.00 |

| Purpose of mixing | Materials | Manufacturer | Component | Ex. 15 | Ex. 16 | Ex. 17 | Ex. 18 | Ex. 19 | Ex. 20 | Comp. Ex. 5 |
|---|---|---|---|---|---|---|---|---|---|---|
| Surfactant | Total composition (%) | | | 0.050 | 0.210 | 0.140 | 0.140 | 0.010 | 0.010 | 0.100 |
| | HLB | | | 9 | 10 | 9 | 10 | 8 | 8 | 4 |
| (Component B)/(Component A) | | | | 10.0 | 2.4 | 3.6 | 3.6 | 50.0 | 50.0 | 5.0 |
| Evaluation | 1. Appearance | | | Clear | Clear | Clear | Clear | Clear | Clear | Opaque |
| | 2. Emulsion type | | | O/W | O/W | O/W | O/W | O/W | O/W | W/O |
| | 3. Readiness of emulsification | | | B | A | A | A | B | A | A |
| | 4. Separability | | | A | B | A | A | A | A | D |
| | 5. Cleansing power | | | B | A | A | A | B | B | B |
| | 6. Feel to the touch upon use | | | B | A | B | B | A | B | B |

EP 2 438 905 B1

All Examples yielded good clearness when separated into multiple layers, O/W-type emulsion when shaken, and excellent levels of readiness of emulsification, separability, cleansing power, and feel to the touch upon use.

In contrast, Comparative Examples were poor in any of the appearance, readiness of emulsification, separability, cleansing power and feel to the touch upon use.

While Example 11 showed a level of separability of B, the level was slightly lower than that of Example 12, due to a smaller content of the component (B) as compared with Example 12.

**Claims**

1. A clear multi-layered liquid cosmetic comprising:

   (A) not less than 0.0005% by weight, and not more than 1% by weight of a nonionic surfactant comprising a single species of nonionic surfactant, or a mixed surfactant containing two or more species of nonionic surfactants mixed therein, having an HLB of the single species of nonionic surfactant or the mixed surfactant of 5 to 11;
   (B) not less than 0.05% by weight, and not more than 5% by weight of a water-soluble cellulose derivative;
   (C) not less than 10% by weight, and not more than 90% by weight of an oil having a viscosity at 30°C of not more than 30 mPa·s; and
   (D) water,

   wherein the cosmetic is configured to separate into multiple layers when allowed to stand still, and to form an O/W-type emulsion when mixed by shaking, and
   wherein the ratio by weight given by (B)/(A) is not less than 2, and not more than 500.

2. The clear multi-layered liquid cosmetic according to Claim 1,
   wherein the content of the component (A) is not less than 0.001% by weight, and not more than 0.29% by weight.

3. The clear multi-layered liquid cosmetic according to Claim 1 or 2,
   wherein the water-soluble cellulose derivative component (B) comprises a single species, or two or more species selected from the group consisting of hydroxyethyl cellulose, hydroxypropyl cellulose, hydroxypropylmethyl cellulose, hydroxyethylmethyl cellulose, and hydroxypropylethyl cellulose.

4. The clear multi-layered liquid cosmetic according to any one of Claims 1 to 3,
   wherein the component (A) comprises a nonionic surfactant having two or more hydroxyl groups.

5. The clear multi-layered liquid cosmetic according to any one of Claims 1 to 4,
   wherein the component (A) comprises a nonionic surfactant having a hydroxyl group and an amide group.

6. The clear multi-layered liquid cosmetic according to any one of Claims 1 to 5,
   wherein the component (A) comprises a nonionic surfactant existing as a liquid or as a paste at 20°C,.

7. The clear multi-layered liquid cosmetic according to any one of Claims 1 to 6, further comprising a single species, or two or more species of water-miscible solvent selected from (E1) to (E3) below:

   (E1) a compound having two or more oxypropylene groups and hydroxyl groups in the molecule thereof, and having a value of (the number of oxypropylene groups/the number of hydroxyl groups) of 5 or smaller;
   (E2) a monohydric alcohol having 2 to 6 carbon atoms ; and
   (E3) a dihydric alcohol having 2 to 6 carbon atoms.

8. The clear multi-layered liquid cosmetic according to any one of Claims 1 to 7,
   wherein the component (A) is not more than 0.02% by weight.

9. The clear multi-layered liquid cosmetic according to any one of Claims 1 to 8,
   wherein the clear multi-layered liquid cosmetic does not contain a surfactant except the component (A).

10. The clear multi-layered liquid cosmetic according to any one of Claims 1 to 9,
    wherein the cosmetic is configured to contain no inorganic salt, or to have a content of inorganic salt of not more than 0.03% by weight.

11. Use of the clear multi-layered liquid cosmetic according to any one of Claims 1 to 10 for removing a makeup by applying it onto a made-up skin after mixing it by shaking.

12. A method of removing makeup materials by applying a clear multi-layered liquid cosmetic, after mixing it by shaking, onto a made-up skin,
wherein the clear multi-layered liquid cosmetic comprises:

(A) not less than 0.0005% by weight , and not more than 1% by weight of a nonionic surfactant comprising a single species of nonionic surfactant, or a mixed surfactant containing two or more species of nonionic surfactants mixed therein, having an HLB of the single species of nonionic surfactant or the mixed surfactant of 5 to 11;
(B) not less than 0.05% by weight , and not more than 5% by weight of a water-soluble cellulose derivative;
(C) not less than 10% by weight , and not more than 90% by weight of an oil having a viscosity at 30°C of not more than 30 mPa·s; and
(D) water,

wherein the cosmetic is configured to separate into multiple layers when allowed to stand still, and to form an O/W-type emulsion when mixed by shaking, and
wherein the ratio by weight given by (B)/(A) is not less than 2, and not more than 500.

**Patentansprüche**

1. Klares, vielschichtiges, flüssiges Kosmetikum, enthaltend:

(A) nicht weniger als 0,0005 Gew.% und nicht mehr als 1 Gew.% eines nicht-ionischen Tensides, enthaltend eine einzelne Spezies von nicht-ionischem Tensid oder ein gemischtes Tensid, enthaltend zwei oder mehr Spezies von nicht-ionischen Tensiden, die darin gemischt sind, mit einem HLB der einzelnen Spezies von nicht-ionischem Tensid oder dem gemischten Tensid von 5 bis 11;
(B) nicht weniger als 0,05 Gew.% und nicht mehr als 5 Gew.% eines wasserlöslichen Celluosederivates;
(C) nicht weniger als 10 Gew.% und nicht mehr als 90 Gew.% eines Öls mit einer Viskosität bei 30°C von nicht mehr als 30 mPa·s, und
(D) Wasser,

worin das Kosmetikum konfiguriert ist, zur Trennung in viele Schichten, wenn es stehengelassen wird, und zur Bildung einer Emulsion vom O/W-Typ, wenn es durch Schütteln gemischt wird, und
worin das Gewichtsverhältnis, gegeben durch (B)/(A), nicht weniger als 2 und nicht mehr als 500 ist.

2. Klares, vielschichtiges, flüssiges Kosmetikum gemäß Anspruch 1, worin der Gehalt der Komponente (A) nicht weniger als 0,001 Gew.% und nicht mehr als 0,29 Gew.% ist.

3. Klares, vielschichtiges, flüssiges Kosmetikum gemäß Anspruch 1 oder 2, worin die wasserlösliche Cellulosederivatkomponente (B) eine einzelne Spezies oder zwei oder mehrere Spezies enthält, ausgewählt aus der Gruppe, bestehend aus Hydroxyethylcellulose, Hydroxypropylcellulose, Hydroxypropylmethylcellulose, Hydroxyethylmethylcellulose und Hydroxypropylethylcellulose.

4. Klares, vielschichtiges, flüssiges Kosmetikum gemäß einem der Ansprüche 1 bis 3, worin die Komponente (A) ein nicht-ionisches Tensid mit zwei oder mehreren Hydroxylgruppen enthält.

5. Klares, vielschichtiges, flüssiges Kosmetikum gemäß einem der Ansprüche 1 bis 4, worin die Komponente (A) ein nicht-ionisches Tensid mit einer Hydroxylgruppe und einer Amidgruppe enthält.

6. Klares, vielschichtiges, flüssiges Kosmetikum gemäß einem der Ansprüche 1 bis 5, worin die Komponente (A) ein nicht-ionisches Tensid enthält, das als Flüssigkeit oder als Paste bei 20°C existiert.

7. Klares, vielschichtiges, flüssiges Kosmetikum gemäß einem der Ansprüche 1 bis 6, weiterhin enthaltend eine einzelne Spezies oder zwei oder mehrere Spezies eines wassermischbaren Lösungsmittels, ausgewählt aus (E1) bis (E3) unten:

(E1) Verbindung mit zwei oder mehreren Oxypropylengruppen und Hydroxylgruppen im Molekül davon und mit einem von Wert (Zahl der Oxypropylengruppen/Zahl der Hydroxylgruppen) von 5 oder kleiner;
(E2) einwertiger Alkohol mit 2 bis 6 Kohlenstoffatomen; und
(E3) zweiwertiger Alkohol mit 2 bis 6 Kohlenstoffatomen.

8. Klares, vielschichtiges, flüssiges Kosmetikum gemäß einem der Ansprüche 1 bis 7, worin die Komponente (A) nicht mehr als 0,02 Gew.% ist.

9. Klares, vielschichtiges, flüssiges Kosmetikum gemäß einem der Ansprüche 1 bis 8, worin das klare, vielschichtige, flüssige Kosmetikum kein Tensid mit Ausnahme der Komponente (A) enthält.

10. Klares, vielschichtiges, flüssiges Kosmetikum gemäß einem der Ansprüche 1 bis 9, worin das Kosmetikum konfiguriert ist, dass es kein anorganisches Salz enthält oder einen Gehalt an anorganischem Salz von nicht mehr als 0,03 Gew% hat.

11. Verwendung des klaren, vielschichtigen, flüssigen Kosmetikums gemäß einem der Ansprüche 1 bis 10, zur Entfernung von Make-up durch Auftragen auf geschminkte Haut nach Mischen durch Schütteln.

12. Verfahren zur Entfernung von Make-up-Materialien durch Auftragen eines klaren, vielschichtigen, flüssigen Kosmetikums nach Mischen durch Schütteln auf eine geschminkte Haut,
worin das klare, vielschichtige flüssige Kosmetikum enthält:

(A) nicht weniger als 0,0005 Gew.% und nicht mehr als 1 Gew.% eines nicht-ionischen Tensides, enthaltend eine einzelne Spezies von nicht-ionischem Tensid oder ein gemischtes Tensid, enthaltend zwei oder mehr Spezies von nicht-ionischen Tensiden, die darin gemischt sind, mit einem HLB der einzelnen Spezies von nicht-ionischem Tensid oder dem gemischten Tensid von 5 bis 11;
(B) nicht weniger als 0,05 Gew.% und nicht mehr als 5 Gew.% eines wasserlöslichen Celluosederivates;
(C) nicht weniger als 10 Gew.% und nicht mehr als 90 Gew.% eines Öls mit einer Viskosität bei 30°C von nicht mehr als 30 mPa·s, und
(D) Wasser,

worin das Kosmetikum konfiguriert ist, zur Trennung in viele Schichten, wenn es stehengelassen wird, und zur Bildung einer Emulsion vom O/W-Typ, wenn es durch Schütteln gemischt wird, und
worin das Gewichtsverhältnis, gegeben durch (B)/(A), nicht weniger als 2 und nicht mehr als 500 ist.

**Revendications**

1. Cosmétique liquide multicouche transparent comprenant :

(A) pas moins de 0,0005 % en poids, et pas plus de 1 % en poids d'un tensioactif non ionique comprenant une espèce unique de tensioactif non ionique, ou un tensioactif mixte contenant deux ou plusieurs espèces de tensioactifs non ioniques mélangées dans celui-ci, ayant un HLB de l'espèce unique de tensioactif non ionique ou du tensioactif mixte de 5 à 11 ;
(B) pas moins de 0,05% en poids, et pas plus de 5 % en poids d'un dérivé de cellulose soluble dans l'eau ;
(C) pas moins de 10 % en poids, et pas plus de 90 % en poids d'une huile ayant une viscosité à 30 °C de pas plus de 30 mPa·s; et
(D) de l'eau,

dans lequel le cosmétique est configuré pour se séparer en plusieurs couches lorsqu'il est laissé immobile, et pour former une émulsion de type H/E lorsqu'il est mélangé par agitation, et
dans lequel le rapport en poids donné par (B)/(A) n'est pas moins de 2, et pas plus de 500.

2. Cosmétique liquide multicouche transparent selon la revendication 1,
dans lequel la teneur du composant (A) n'est pas moins de 0,001 % en poids, et pas plus de 0,29 % en poids.

3. Cosmétique liquide multicouche transparent selon la revendication 1 ou 2,
dans lequel le composant dérivé de cellulose soluble dans l'eau (B) comprend une espèce unique, ou deux espèces ou plus sélectionnées dans le groupe constitué de l'hydroxyéthylcellulose, l'hydroxypropylcellulose, l'hydroxypropylméthylcellulose, l'hydroxyéthylméthylcellulose, et l'hydroxypropyléthylcellulose.

4. Cosmétique liquide multicouche transparent selon l'une quelconque des revendications 1 à 3, dans lequel le composant (A) comprend un tensioactif non ionique ayant deux groupes hydroxyles ou plus.

5. Cosmétique liquide multicouche transparent selon l'une quelconque des revendications 1 à 4, dans lequel le composant (A) comprend un tensioactif non ionique ayant un groupe hydroxyle et un groupe amide.

6. Cosmétique liquide multicouche transparent selon l'une quelconque des revendications 1 à 5, dans lequel le composant (A) comprend un tensioactif non ionique existant en tant que liquide ou en tant que pâte à 20 °C.

7. Cosmétique liquide multicouche transparent selon l'une quelconque des revendications 1 à 6, comprenant en outre une espèce unique, ou deux espèces ou plus de solvant miscible à l'eau sélectionnées parmi (E1) à (E3) ci-dessous :

   (E1) un composé ayant deux groupes oxypropylène et groupes hydroxyle ou plus dans la molécule de celui-ci, et ayant une valeur du (nombre de groupes oxypropylène / nombre de groupes hydroxyle) de 5 ou moins ;
   (E2) un alcool monohydrique ayant de 2 à 6 atomes de carbone ; et
   (E3) un alcool dihydrique ayant de 2 à 6 atomes de carbone.

8. Cosmétique liquide multicouche transparent selon l'une quelconque des revendications 1 à 7, dans lequel le composant (A) n'est pas plus de 0,02 % en poids.

9. Cosmétique liquide multicouche transparent selon l'une quelconque des revendications 1 à 8, dans lequel le cosmétique liquide multicouche transparent ne contient pas de tensioactif sauf le composant (A).

10. Cosmétique liquide multicouche transparent selon l'une quelconque des revendications 1 à 9, dans lequel le cosmétique est configuré pour ne contenir aucun sel inorganique, ou pour avoir une teneur en sel inorganique de pas plus de 0,03 % en poids.

11. Utilisation du cosmétique liquide multicouche transparent selon l'une quelconque des revendications 1 à 10 pour enlever un maquillage en l'appliquant sur une peau maquillée après son mélange par agitation.

12. Procédé pour enlever des matières de maquillage en appliquant un cosmétique liquide multicouche transparent, après son mélange par agitation, sur une peau maquillée, dans lequel le cosmétique liquide multicouche transparent comprend :

   (A) pas moins de 0,0005 % en poids, et pas plus de 1 % en poids d'un tensioactif non ionique comprenant une espèce unique de tensioactif non ionique, ou un tensioactif mixte contenant deux espèces ou plus de tensioactifs non ioniques mélangées dans celui-ci, ayant un HLB de l'espèce unique de tensioactif non ionique ou du tensioactif mixte de 5 à 11 ;
   (B) pas moins de 0,05% en poids, et pas plus de 5 % en poids d'un dérivé de cellulose soluble dans l'eau ;
   (C) pas moins de 10 % en poids, et pas plus de 90 % en poids d'une huile ayant une viscosité à 30 °C de pas plus de 30 mPa·s ; et
   (D) de l'eau,

   dans lequel le cosmétique est configuré pour se séparer en couches multiples lorsqu'il est laissé immobile, et pour former une émulsion de type H/E lorsqu'il est mélangé par agitation, et
   dans lequel le rapport en poids donné par (B)/(A) n'est pas moins de 2, et pas plus de 500.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H0899836 A
- JP H10101529 A
- JP 2004203764 A
- JP H11246348 A
- JP 2004217641 A
- WO 0215849 A